# EUROPEAN PATENT APPLICATION

(11) **EP 4 825 302 A1**
(43) Date of publication of application: **21.10.2026**
(21) Application number: 25170551.3
(22) Date of filing: 15.04.2025
(51) Int. Cl.: A61B 1/00, A61B 1/015, A61B 10/00

(54) **MEDICAL SAMPLING DEVICE AND SYSTEM COMPRISING THE MEDICAL SAMPLING DEVICE**

(71) Applicant: Ambu A/S, 2750 Ballerup (DK)
(72) Inventor: AL ATRAKTCHI, Abbas, 2300 Copenhagen S (DK)
(74) Representative: Winter, Brandl - Partnerschaft mbB

(57) **Abstract**

The present disclosure relates to a medical sampling device (2) and a system comprising the medical sampling device (2). The medical sampling device (2) is attachable to a suction connector (3) of an endoscope (4), to a sample container (6) and to a suctioning source (13). The medical sampling device (2) is operable in at least a sampling-mode and a bypass-mode, the sampling-mode being a sample-collection-mode in which a specimen sample from the endoscope (4) is collected in the sample container (6), the bypass-mode being a suctioning-mode in which the specimen sample is directly guided towards the suctioning source, thereby bypassing the sample container (6). The medical sampling device (2) comprises a pinch valve (8) having at least a soft tubing unit (10) and a pinching unit (12), the pinching unit (12) being configured to pinch the soft tubing unit (10) in at least a first pinching state providing the sampling-mode and a second pinching state providing the bypass-mode.

## Description

The present disclosure relates to a medical sampling device, in particular a sampler bridge, and a system comprising the medical sampling device, wherein the medical sampling device is attachable to a suction connector of an endoscope, to a sample container, and to a suctioning source, wherein the medical sampling device is operable in at least a sampling-mode and a bypass-mode, the sampling-mode being a sample-collection-mode in which a specimen sample from the endoscope is collected in the sample container, and the bypass-mode being a suctioning-mode in which the specimen sample is directly guided towards the suctioning source, thereby bypassing the sample container.

### Related Art

Endoscopes which include specialized instruments such as bronchoscopes are well known from the related art and are used for visual examination and diagnosis of hollow organs and body cavities, as well as to assist in surgery, e.g. for a targeted tissue sampling. Both reusable and disposable (i.e. single-use) endoscopes are known from the related art. Known endoscopes usually comprise an endoscope handle via which an operator can hold and control the endoscope. An insertion cord comprising an insertion tube, an actively bendable bending section, which is a section of increased flexibility, and a distal tip unit is usually connected to the endoscope handle. The insertion cord is configured to be inserted into the hollow organs and body cavities of a patient.

A typical procedure which is performed with a bronchoscope or in other words during a bronchoscopy is a bronchoalveolar lavage (BAL). BAL is used to collect a sample of fluid from the lower respiratory tract, specifically the bronchioles and alveoli, for diagnostic or research purposes. To perform this bronchoscopic procedure, a bronchoscope is guided into a specific region of the lung, a sterile saline solution is instilled into the lung segment, and the fluid is gently suctioned back, collecting cells, microorganisms, proteins and other components from the alveolar space.

The endoscopic procedure may involve suctioning fluids without sampling them. For example, body fluids may be suctioned to enable a camera of the endoscope to obtain usable images within the body cavity or to remove debris such as mucus or blood. Therefore, it is preferable to provide a valve configured to direct the fluid mixture suctioned through the endoscope to a sample container or to bypass the sample container and direct the fluid mixture to a waste container.

In order to be able to perform the BAL procedure, there are already known broncho sampler sets comprising a medical sampling device, in particular a sampler bridge, an endoscope, in particular a bronchoscope, and a sample container, wherein the medical sampling device is attachable to the endoscope and to the sample container. The medical sampling device is operable in a sampling-mode and a bypass-mode, wherein the sampling-mode is a sample-collection-mode in which a specimen sample is collected in the sample container, and the bypass-mode is a suctioning-mode in which the specimen sample is directly guided towards a suctioning source, thereby bypassing the sample container.

To reduce labor and complexity, it is preferred that one operator handles the endoscope, the medical sampling device, and the sample container. For this purpose, the medical sampling device may serve to secure the sampling container to the endoscope in a fixed and rigid manner. The medical sampling device should be as small as possible so that the system comprising the endoscope, the sampling device, and the sample container does not become unwieldy to handle by the operator. Additionally, the sampling device should minimize fluid pressure drop so that the vacuum provided by a vacuum source connected to the sampling device, and through the sampling device to the working channel valve, is fully used to suction the fluid mixture from the patient. Full use of the vacuum will reduce the duration of the endoscopic procedure by suctioning fluids more quickly than if less vacuum were used. Additionally, since the endoscope and the patient may move, it is desirable, when the tip of the endoscope is in the desired position, to withdraw the samples quickly before any movement dislodges the tip of the endoscope from the desired position. Even further, it is desirable to withdraw the samples quickly before any body fluids refill the space where the samples are to be taken. These requirements contravene since reduced size limits the space available to provide fluid channels, which should be as large as possible to reduce the fluid pressure drop.

### Brief description of the disclosure

The tasks and objectives of the present disclosure are to eliminate or at least to reduce the disadvantages of the related art. In particular, a medical sampling device shall be provided that reduces the pressure loss through the medical sampling device, has a simple design and can be easily assembled.

The tasks and objectives are solved by a medical sampling device in accordance with claim 1 and by a system in accordance with claim 14. Advantageous embodiments are claimed in the dependent claims and/or are explained herein below.

The present disclosure relates to a medical sampling device, in particular a sampler bridge, and a system comprising the medical sampling device, wherein the medical sampling device is attachable to a suction connector of an endoscope, preferably a bronchoscope, to a sample container and to a suctioning source, wherein the medical sampling device is operable in at least a sampling-mode and a bypass-mode, the sampling-mode being a sample-collection-mode in which a specimen sample from the endoscope is collected in the sample container, the bypass-mode being a suctioning-mode in which the specimen sample is directly guided towards the suctioning source, thereby bypassing the sample container, the medical sampling device further comprising a pinch valve having at least a soft tubing unit and a pinching unit, the pinching unit being configured to pinch the soft tubing unit in at least a first pinching state providing the sampling-mode and a second pinching state providing the bypass-mode.

In other words, the present disclosure provides a medical sampling device having, inter alia, a pinch valve enabling the bypass-mode and the sampling-mode by suitably pinching the soft tubing unit with the pinching unit. The disclosed pinch valve provided within the medical sampling device advantageously offers a simple pinching solution, via which internal fluid flow can be suitably adjusted, in particular reliably prevented via a pinching engagement between the pinching unit and the soft tubing unit at desired positions, thereby realizing at least the sampling-mode and the bypass-mode. It has turned out that the pinching engagement leads to a reduced pressure loss both in the first pinching state providing the sampling-mode and in the second pinching state providing the bypass-mode. A benefit is that the soft tubing unit may be fluidly connected to other flow channels with no need for separate sealings and no disconnection of flow channels, so the risk of leaks is reduced or even eliminated. Such leaks meaning that biomaterial escape could otherwise constitute a potential risk to persons and increase risk of infection.

It is added that the endoscope to which the medical sampling device is attachable may be a flexible endoscope or a rigid endoscope. The endoscope may be a reusable or a single-use endoscope. Preferably, the endoscope is a single-use endoscope. Especially preferred also the medical sampling device and the sample container are single use devices or components. Further, the endoscope is preferably a bronchoscope, however may alternatively also be a gastroscope, a colonoscope, a laparoscope, an arthroscope, a cystoscope, a hysteroscope, etc.

Since the medical sampling device is attachable to the endoscope, which is preferably a bronchoscope, and to the sample container, it is easy to perform a bronchoalveolar lavage (BAL) during a bronchoscopic procedure, in particular when the medical sampling device is also attachable to a suction source enabling fluid flow and suctioning both in the bypass-mode and the sampling-mode.

Furthermore, since the sample container is attachable and detachable from the medical sampling device, the sample container can be exchanged during usage after switching from the sampling-mode to the bypass-mode. An exchangeable sample container in a set or system comprising at least the medical sampling device and the sample container, preferably also the endoscope, offers the advantage that a desired probe volume is not restricted by a volume of the sample container. The aforementioned modularity may improve workflow efficiency, since multiple samples can be collected sequentially without interrupting the endoscopic, in particular bronchoscopic, procedure.

Additionally, an exchangeable sample container enables precise sample handling and storage, reducing the risk of contamination or degradation before laboratory analysis. In research and diagnostic settings, this feature may support higher throughput and consistency, making the system or set with the disclosed medical sampling device more versatile and cost-effective.

The bypass-mode and the sampling-mode in such a system or set enable an appropriate sample collection in the sample container in the sampling-mode, without interrupting suction when the sample container is full, since a user can then easily and reliably switch to the bypass-mode. This offers advantages in both clinical and procedural efficiency. By maintaining continuous suctioning, uninterrupted operation is ensured. The seamless transition between containers enhances procedural workflow, eliminating the need for system pauses or manual interventions, which can prolong the examination or treatment. Additionally, the uninterrupted suction reduces patient discomfort by minimizing procedural delays and ensuring a smoother, more efficient experience. This advantageously enhances user control, improves sample integrity and contributes to a more patient-friendly and hygienic endoscopic procedure.

Furthermore, by using the disclosed medical sampling device having the pinch valve providing the pinching engagement in at least two pinching states between the pinching unit and the soft tubing unit, the pressure loss in the medical sampling device can be reduced, allowing consistent suction in the sampling-mode and the bypass-mode. This further enhances procedural efficiency and reduces patient discomfort.

The medical sampling device may comprise a sample container connection interface adapted/ designed for connection to the sample container. In particular, the medical sampling device may comprise a sample container attachment element or portion, on which the sample container connection interface is formed. The sample container connection interface is to be understood as direct or immediate interface or contact point, where the sample container is attached to the medical sampling device. The sample container connection interface may be provided on a separate sample container attachment element or part. Alternatively, the sample container connection interface may be provided on a portion of another part or component of the medical sampling device, i.e. may be provided on a sample container attachment portion of another part or component of the medical sampling device.

The medical sampling device may comprise an endoscope connection interface adapted/ designed for connection to the endoscope. In particular, the medical sampling device may comprise an endoscope attachment element or portion, on which the endoscope connection interface is formed. The endoscope connection interface is to be understood as direct or immediate interface or contact point, where the endoscope is attached to the medical sampling device. The endoscope connection interface may be provided on a separate endoscope attachment element or part. Alternatively, the endoscope connection interface may be provided on a portion of another part or component of the medical sampling device, i.e. may be provided on an endoscope attachment portion of another part or component of the medical sampling device.

The medical sampling device may comprise a suctioning source connection interface adapted for connection to a suctioning source. In particular, the medical sampling device may comprise a suctioning source attachment element or portion, on which the suctioning source connection interface is formed. The suctioning source connection interface is to be understood as direct or immediate interface or contact point, where a suctioning source or a tube or hose leading to the suctioning source is attached. The suctioning source connection interface may be provided on a separate suctioning source attachment element or part. Alternatively, the suctioning source connection interface may be provided on a portion of another part or component of the medical sampling device, i.e. may be provided on a suctioning source attachment portion of another part or component of the medical sampling device.

The medical sampling device may comprise a first axial end on which the endoscope is attachable and a second axial end (opposite the first axial end) on which the sample container is attachable. Of course, the endoscope is preferably also detachable from the first axial end, and the sample container is preferably also detachable from the second axial end. It is to be understood that the endoscope and the sample container are not necessarily arranged opposite to each other on opposing axial ends according to the present disclosure. For example, it is also possible to connect the endoscope or the sample container substantially perpendicularly to each other.

According to a preferred embodiment, the endoscope connection interface is provided on the first axial end, the sample container connection interface is provided on the second axial end, and a longitudinal axis is formed between the first axial end and the second axial end.

The suctioning source connection interface may be oriented at an angle with respect to the longitudinal axis, preferably at a right angle.

Preferably, the medical sampling device comprises a housing, in which the pinch valve is disposed.

The pinching unit of the pinch valve may be an integral part or may comprise a plurality of pinching parts.

The pinch valve may comprise an actuation unit configured to actuate the pinching unit, in particular to bring the pinching unit in at least the first pinching state and the second pinching state. Preferably, the actuation unit is a mechanical actuation unit, in particular is or comprises a rotatable or displaceable actuation element or part or portion, e.g. a lever or switch or wheel part or portion. Alternatively, the actuation unit may be an electrical actuation unit, which e.g. comprise an electrical switch, a control unit and an electric motor rotating or translating the pinching unit.

The pinch valve may comprise a tubing holder configured and provided to hold the soft tubing unit.

The soft tubing unit may be an integral part or may comprise a plurality of tubes.

The soft tubing unit may comprise a first fluid connection having a first endoscope end and a first sample container end, and a second fluid connection having a second endoscope end, a second sample container end and a suctioning source end. The second fluid connection may have an endoscope portion, a sample container portion and a suctioning source portion, each extending from a common connection point, i.e. the second fluid connection may essentially be formed in a T-shape.

The pinching unit may be configured to pinch the soft tubing unit in the second pinching state providing the bypass-mode such that fluid flow or suctioning is enabled between the second endoscope end and the suctioning source end. In particular, the pinching unit may be configured to pinch the soft tubing unit in the second pinching state providing the bypass-mode at a first position on the first fluid connection, the first position may be an arbitrary position on the first fluid connection, thereby preventing fluid flow via the first fluid connection. Further, the pinching unit may be configured to pinch the soft tubing unit in the second pinching state providing the bypass-mode at a second position on the second fluid connection, the second position may be a position downstream of the connection point on the second fluid connection, thereby enabling fluid flow between the second endoscope end and the suctioning source end and preventing fluid flow towards and from the second sample container end.

The pinching unit may be configured to pinch the soft tubing unit in the first pinching state providing the sampling-mode such that fluid flow or suctioning is enabled from the first endoscope end to the first sample container end, via the sample container to the second sample container end and from the second sample container end to the suctioning source end. In particular, the pinching unit may be configured to pinch the soft tubing unit in the first pinching state providing the sampling-mode such that fluid flow is prevented between the second endoscope end and the suctioning source end. Further, the pinching unit may be configured to pinch the soft tubing unit in the first pinching state providing the sampling-mode at a third position on the second fluid connection, the third position may be a position upstream of the connection point on the second fluid connection, thereby preventing fluid flow between the second endoscope end and the second sample container end.

According to an embodiment, the pinching unit may be a rotating part having an axis of rotation corresponding to the longitudinal axis.

Further, according to an embodiment, the pinching unit may be a rotating part having an axis of rotation arranged essentially perpendicularly to the longitudinal axis.

Furthermore, according to an embodiment, the pinching unit may be formed as the rotating part and may comprise a first engagement end or portion (e.g. a wide clamping portion) configured and provided to pinch the first fluid connection and the second fluid connection, and a second engagement end or portion (e.g. a narrow clamping portion) configured and provided to pinch only the second fluid connection (i.e. not the first fluid connection).

Preferably, the first engagement end or portion has an engagement length greater than an engagement length of the second engagement end or portion.

Furthermore, according to an embodiment, the first engagement end or portion may be arranged essentially diametrically opposed to the second engagement end or portion.

According to an embodiment, the pinching unit formed as the rotating part, in the first pinching state providing the sampling-mode, may be rotated in(to) a first position in which the second engagement end pinches the second fluid connection.

Furthermore, the pinching unit formed as the rotating part in the second pinching state providing the bypass-mode may be rotated in a second position in which the first engagement end pinches the first fluid connection and the second fluid connection.

The medical sampling device may have a locking or fixing mechanism, in particular a snap-in mechanism, configured and provided to lock or fixate the pinching unit in the first pinching state and/or in the second pinching state.

The locking or fixing mechanism, in particular the snap-in mechanism, may be formed by a protruding (elastic or spring) portion, preferably of the pinching unit, in particular of the rotating part, engaging in a recessed portion, preferably of a stationary part like the housing or the tubing holder.

According to an aspect of the disclosure, an additional locking mechanism is not necessary, if the internal friction between the rotating part and the stationary part is set to prevent elastic restoring displacements of the pinched tubing unit. Moreover, a separate fixing mechanism is also not necessary according to another aspect of the disclosure, when electrical components are used to rotate the rotating part, since a motor inherently has a stopping effect. This is due to the fact that when a power to the motor is cut off, it naturally ceases movement due to an internal resistance, friction, or the way the motor is designed to stop. Therefore, the built-in stopping mechanism of the motor may eliminate the need for an additional snap mechanism to halt its motion.

It has been found that silicone, TPU and rubber are preferable materials for medical pinched tubes due to their flexibility, durability and biocompatibility. Silicone is highly resistant to temperature extremes, while TPU offers excellent elasticity and chemical resistance. Rubber provides resilience and reliable sealing.

The present disclosure further relates to a system comprising the medical sampling device according to one of the above aspects, and a sample container, preferably also an endoscope.

The system may further comprise a suctioning source (device) that is connectable directly or indirectly, for example through a tube or hose, to the suctioning source connection interface.

The suctioning source may be a vacuum pump or the like. There are also embodiments imaginable, in which the suctioning source (device) is directly coupled to the endoscope or is part of the endoscope.

### Brief description of the figures

The disclosure is explained in more detail below using preferred embodiments and referring to the accompanying figures.
Fig. 1 shows an exploded perspective view of a system comprising a medical sampling device according to a first embodiment, an endoscope and a sample container.
Fig. 2 shows a perspective view of the medical sampling device according to the first embodiment.
Fig. 3 shows an exploded view of the medical sampling device according to the first embodiment.
Fig. 4 shows a perspective view of a sample container connection interface of the medical sampling device according to the first embodiment.
Fig. 5 shows a perspective view of an endoscope connection interface of the medical sampling device according to the first embodiment.
Fig. 6 shows a perspective view of a housing accommodating a pinch valve of the medical sampling device according to the first embodiment.
Fig. 7 shows a perspective view of a tubing holder of the medical sampling device according to the first embodiment.
Fig. 8 shows a perspective view of a pinching unit of the medical sampling device according to the first embodiment.
Fig. 9 shows a perspective view of a soft tubing unit of the medical sampling device according to the first embodiment.
Fig. 10 shows a perspective view of a pinch valve of the medical sampling device according to the first embodiment in a sampling-mode.
Fig. 11 shows a perspective view of the pinch valve of the medical sampling device according to the first embodiment in a bypass-mode.
Fig. 12 shows an exploded cross sectional view of the medical sampling device according to the first embodiment with illustrated pinching positions.
Fig. 13 shows a perspective view of the soft tubing unit of the medical sampling device according to the first embodiment with illustrated pinching positions in the sampling mode.
Fig. 14 shows a perspective view of the soft tubing unit of the medical sampling device according to the first embodiment with illustrated pinching positions in the by-pass mode.
Fig. 15 shows a perspective view of a medical sampling device according to a second embodiment.
Fig. 16 shows an exploded perspective view of the medical sampling device according to the second embodiment.
Fig. 17 shows a schematic illustration of a pinch valve prototype of a medical sampling device according to a third embodiment.
Fig. 18 shows a schematic illustration of a pinch valve prototype of a medical sampling device according to a fourth embodiment in a sampling-mode.
Fig. 19 corresponds to Fig. 18 but with the pinch valve in bypass-mode.
Fig. 20 shows a perspective view of the medical sampling device according to the fourth embodiment.
Fig. 21 shows a sectional view of a snap-in mechanism.

The figures are schematic in nature and serve only to understand the disclosure. The features of the different embodiments can be interchanged among each other.

### Detailed description of the figures

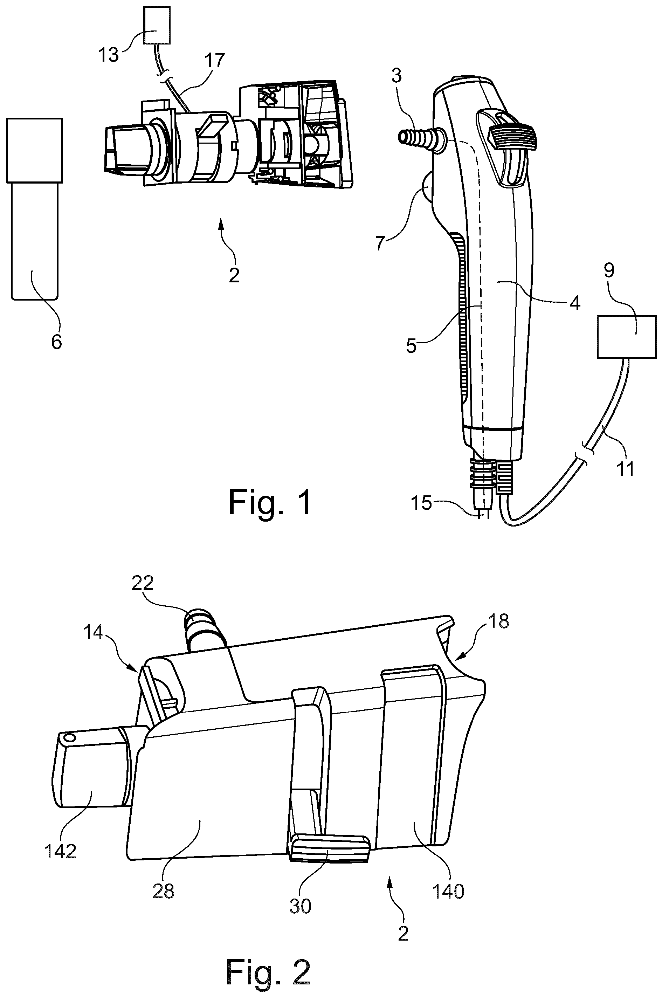

Fig. 1 is an exploded perspective view of a sampling system or set comprising a medical sampling device 2 according to a first embodiment, an endoscope 4 and a sample container 6. The medical sampling device 2 is connectable to the sample container 6 and the endoscope 4. The endoscope 4 comprises an insertion cord 15 (only shown in part) having a proximal end connected to a handle of the endoscope and a distal end insertable in a body cavity of a patient. A working channel 5 runs through the insertion cord to an opening at the distal end of the insertion cord. At the other end the working channel 5 may continue inside the handle as illustrated and end at a suction connector 3. The suction connector 3 may be directly or indirectly connected to a suctioning source 13. In the illustrated example the suctioning source 13 is fluidly connected via a hose 17 to the medical sampling device 2, which in turn is ready for attachment to the suction connector 3 of the endoscope 4 for fluidly connecting to the working channel 5 of the endoscope 4. Suction through the endoscope may be controlled by a valve (not shown) operated by a suction button 7. The sample container 6 may in turn be connected to the medical sampling device 2. The endoscope distal tip may further include a light source and a camera connectable to a video processing apparatus and a monitor 9 via a cable 11 allowing the operator to monitor the interior of the body cavity and activities performed.

Fig. 2 is a perspective view of the medical sampling device 2 according to the first embodiment. The medical sampling device 2 is in a fully assembled state and therefore compact and aesthetical appealing. In the illustrated assembled state, a housing 28, a sample container connection interface 14, an endoscope connection interface 18, a suction source connection interface 22, a mechanical actuation unit 30, a locking key 140 and a cover 142 for the sample container connection interface 14 are visible. When engaged and fully assembled (i.e. when the endoscope 4 is connected to the endoscope connection interface 18), the locking key 140 locks the medical sampling device 2 to the endoscope 4 in a fixed position. The mechanical actuation unit 30 comprises an L-shaped fluted section, which is adapted to be easily touched/ gripped by the user in an ergonomic way. Further, the suction source connection interface 22 is barbed in order to securely connect to a hose. As can be seen, the cover 142 fully sheathes fluid openings of the sample container interface 14, to safeguard them from external contamination and other possible unintended influences.

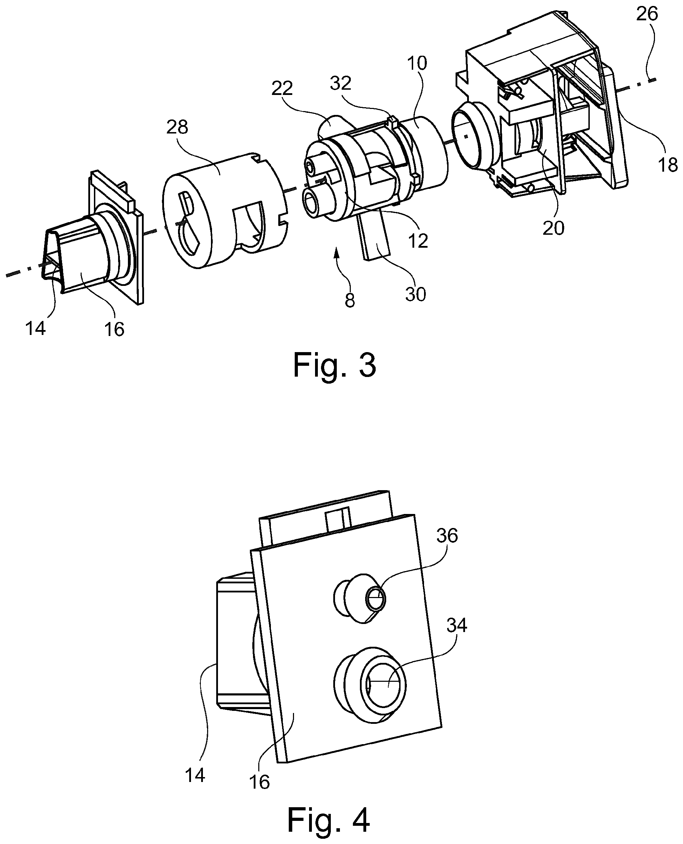

Fig. 3 is an exploded view of the medical sampling device 2 according to the first embodiment. The medical sampling device 2 comprises the sample container connection interface 14 provided on a sample container attachment element 16, the endoscope connection interface 18 provided on an endoscope attachment element 20, the housing 28 and a pinch valve 8, which is accommodated in the housing 28. The pinch valve 8 comprises the suctioning source interface 22. The sample container attachment element 16, the housing 28, the pinch valve 8 and the endoscope attachment element 20 are all arranged along a longitudinal axis 26.

The pinch valve 8 comprises a soft tubing unit 10, which is pinchable by a pinching unit 12, and a tubing holder 32, which is configured and provided to hold the soft tubing unit 10. Further, the pinching unit 12 has the mechanical actuation unit 30, which is formed as a lever.

A sampling-mode and a bypass-mode can be achieved by rotating the pinching unit 12, in particular the mechanical actuation unit 30 in form of the lever, around a rotational axis, which substantially corresponds to the longitudinal axis 26 in the first embodiment of the medical sampling device 2. A rotation of the mechanical actuation unit 30, i.e. of the pinching unit 12, leads to a rotational movement of the pinching unit 12 relative to the tubing holder 32 and the housing 28. By rotating the pinching unit 12 with respect to the housing 28 and the tubing holder 32, the soft tubing unit 10 is pinched in predetermined positions. The pinching of the soft tubing unit 10 in the predetermined positions defines the respective modes, namely the sampling-mode and the bypass-mode. Preferably, the pinching unit 12, in particular the mechanical actuation unit 30, is rotated or rotatable around 90° in order to switch between the sampling-mode and the bypass-mode. The medical sampling device 2 preferably allows just this one internal degree of freedom, which is the rotation of the pinching unit 12 around the longitudinal axis 26. Pinching may be effected for example by squeezing or bending the tube to close the inner opening and cut the flow.

Further, the components or parts of the medical sampling device 2 can be divided into a stationary group and a rotatable group. The stationary group comprises the tubing holder 32, the soft tubing unit 10, the housing 28, the sample container attachment element 16 and the endoscope attachment element 20. The rotatable group comprises the pinching unit 12. It is added that the system borders of the internal degrees of freedom do not include external degrees of freedom, such as movements of the entire medical sampling device 2 or the entire set comprising the medical sampling device 2, the endoscope 4 and the sample container 6, e.g. during use, assembly, disassembly, etc. and movements of connection elements, which occur when the endoscope 4 is connected or disconnected, or when the sample container 6 is connected or disconnected, for example.

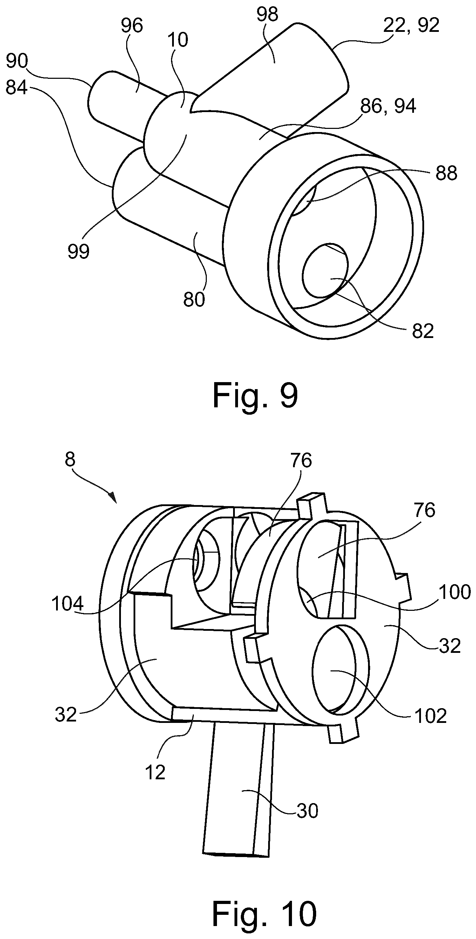

Fig. 4 shows a perspective view of the sample container attachment element 16 having the sample container connection interface 14 according to the medical sampling device 2 of the first embodiment of the present disclosure. The sample container attachment element 16 comprises a first tubing connection 34 and a second tubing connection 36, which are configured to connect to the soft tubing unit 10 (see Fig. 9). The sample container attachment element 16, in particular the sample container connection interface 14 is further configured to connect to the sample container 6 in a safe manner.

Preferably, the connections of the first tubing connection 34 and the second tubing connection 36 are achieved by barbed fittings or connectors, which enable a fluid connection between the sample container 6 and the soft tubing unit 10 while additionally providing fluid-impermeable sealings, in particular when the soft tubing unit 10 is connected to the first and second tubing connections 34, 36 of the sample container attachment element 16, for a safe separation of the liquid from the environment and vice versa.

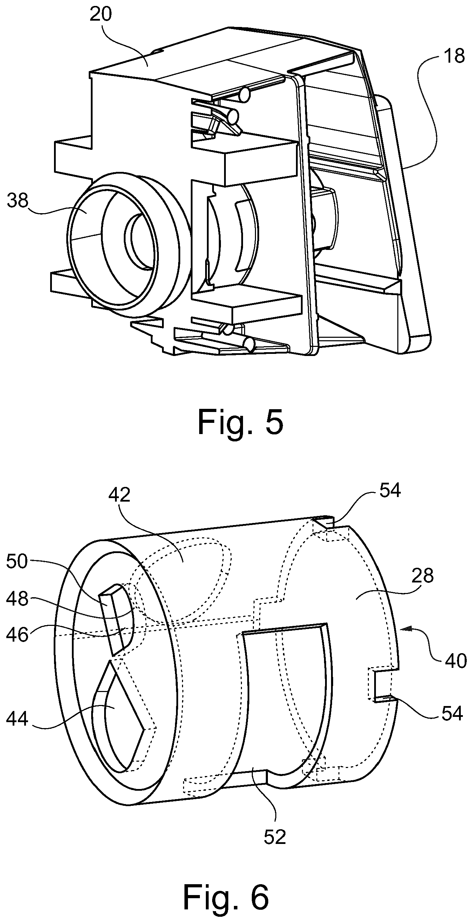

Fig. 5 shows a perspective view of the endoscope attachment element 20 having the endoscope connection interface 18 according to the medical sampling device 2 of the first embodiment of the present disclosure. The endoscope attachment element 20 comprises a tubing connection 38, which is configured to connect to the soft tubing unit 10.

Preferably, the connection of the tubing connection 38 is achieved by a barbed fitting or connector, which enables a fluid connection between the endoscope 4 and the soft tubing unit 10, in particular between the endoscope attachment element 20 and the soft tubing unit 10, while additionally providing fluid-impermeable sealings, in particular when the soft tubing unit 10 is connected to the tubing connection 38, for a safe separation of the liquid from the environment and vice versa. Alternatively, the soft tubing unit could be configured for attachment directly to the endoscope, such as directly to the suction connector 3.

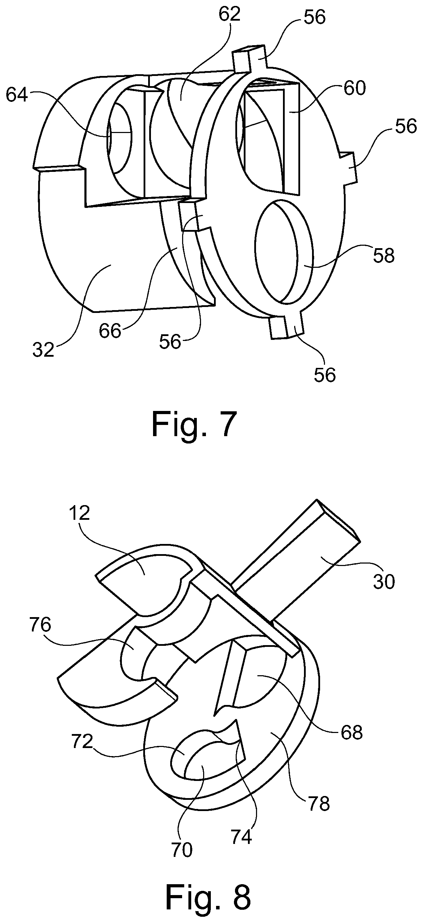

Fig. 6 shows a perspective view of a housing 28 according to the first embodiment of the medical sampling device 2. The housing 28 comprises a housing endoscope opening 40, a housing suctioning source opening 42, a first housing sample container opening 44, a second housing sample container opening 46, an actuation unit opening 52 and preferably a rotational lock to hinder mutual rotation of the housing 28 in relation to the tubing holder 32, here in the form of four tubing holder recesses 54, as will be described in relation to Fig. 7 below.

The first housing sample container opening 44 and the second housing sample container opening 46 each comprise a curved portion 48 and a straight portion 50. The straight portion 50 forms a hold or support for the tube when pinching. The first housing sample container opening 44 is bigger than the second housing sample container opening 46.

The actuation unit opening 52 preferably is a rectangular cutout in a cylindrical wall of the housing 28, further preferably configured to allow substantially a 90° rotation (angle) of the mechanical actuation unit 30, in an assembled state of the medical sampling device 2.

Fig. 7 shows a perspective view of a tubing holder 32 according to the first embodiment of the medical sampling device 2. The tubing holder 32 comprises preferably four tubing holder protrusions 56, which are configured as mating protrusions corresponding to the tubing holder recesses 54 of the housing 28 to form a rotational lock between the housing 28 and the tubing holder 32.

Further, the tubing holder 32 comprises a first tubing holder endoscope opening 58, a second tubing holder endoscope opening 60, a tubing holder suctioning source opening 62, a first tubing holder sample container opening (not shown), a second tubing holder sample container opening 64 and a clamping unit recess 66.

Fig. 8 shows a perspective view of the pinching unit 12 according to the first embodiment of the medical sampling device. The pinching unit 12 comprises a first clamping portion 68, a second clamping portion 70 and a clamping claw 76.

The first clamping portion 68 and the second clamping portion 70 each comprise a curved portion 72 and a straight portion 74.

The clamping claw 76 is configured to be accommodated in the clamping unit recess 66 as shown in Fig. 7 when assembled.

Moreover, the pinching unit 12 comprises the manual actuation unit 30, which has the form of a lever, and which may be mounted to the pinching unit 12 after the parts are assembled.

Fig. 9 shows a perspective view of a soft tubing unit 10 according to the first, second or fourth embodiment of the medical sampling device 2. The soft tubing unit 10 is an integral part and comprises a first fluid connection 80 having a first endoscope end 82 and a first sample container end 84 and a second fluid connection 86 having a second endoscope end 88, a second sample container end 90 and a suctioning source end 92.

Further, the second fluid connection 86 of the soft tubing unit 10 comprises an endoscope portion 94, a sample container portion 96 and a suctioning source portion 98, which combined preferably form a T-shape. These three portions come together at, or in other words extend from a common point 99.

When the medical sampling device 2 is in an assembled state (as illustrated in Fig. 1) comprising the soft tubing unit 10 (Fig. 9), the housing 28 (Fig. 6), the tubing holder 32 (Fig. 7) and the pinching unit 12 (Fig. 8), the first fluid connection 80 (or sample container inlet channel) is configured to fluidly connect the endoscope 4 with the sample container 6 and the second fluid connection is configured to fluidly connect the endoscope 4, the sample container 6 and a suctioning source (not shown). Thereby, the first fluid connection 80 passes through the housing endoscope opening 40, the first tubing holder endoscope opening 58, the first tubing holder sample container opening (not shown), the first clamping portion 68 and the first housing sample container opening 44. Furthermore, the second fluid connection 86 passes through the housing endoscope opening 40, the second tubing holder endoscope opening 60, the clamping claw 76, suctioning source opening 62, the second tubing holder sample container opening (64), the second clamping portion 70 and the second housing sample container opening 46.

Fig. 10 shows a perspective view of part of the pinch valve 8 in a sampling-mode. The soft tubing unit 10 is omitted as well as the housing 28 for better showing the pinching functionality. As can be seen, the passage 100 is closed by the clamping claw 76, which pinches the soft tubing unit 10 in a third pinching position within the endoscope portion 94. Consequently, the second fluid connection 86 is fully closed within the endoscope portion 94, just allowing fluid flow through the first fluid connection 80, the sample container portion 96, which lies within the open passage 104, and the suctioning source portion 98. In other words, no fluid can flow from the endoscope 6, directly to the suctioning source (not shown), while bypassing the sample container 6.

The closed passage 100 is formed, when a corresponding curved portion of the pinching unit 12 moves closer to the corresponding curved portion of the tubing holder 32 by the rotation of the pinching unit 12, thereby clamping the soft tubing unit 10. Due to the omitted soft tubing unit 10, it can be seen that the opening left in the closed passage 100 is oval shaped, or rather pointed oval or almond-shaped.

When the pinch valve 8 is in an assembled state, then a combined first and second position portion 78 of the pinching unit 12 lies outside the tubing holder 32. In other words, an inner surface of the combined first and second position portion 78 abuts against an outer surface of the tubing holder 32.

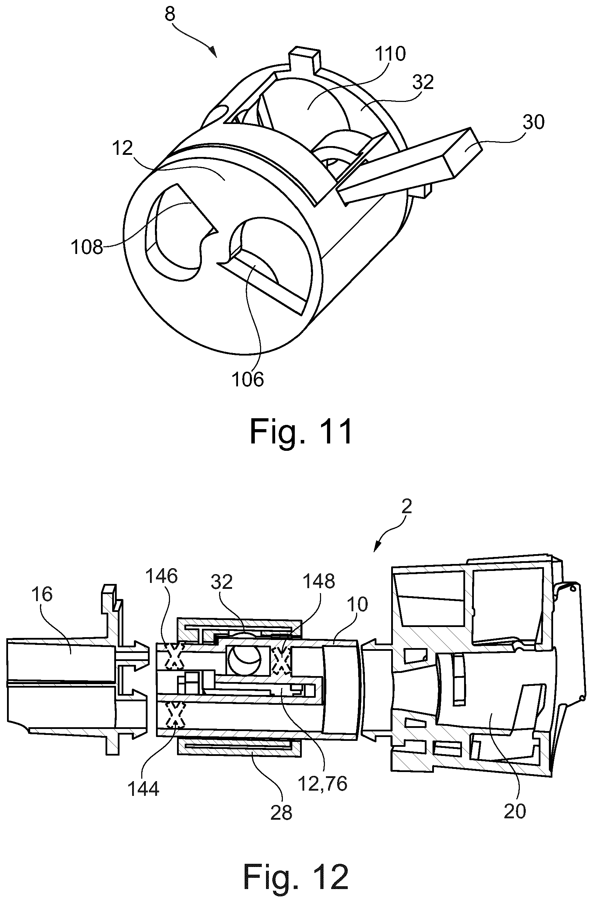

Fig. 11 shows a perspective view of part of the pinch valve 8 in a bypass-mode. The soft tubing unit 10 as well as the housing 28 is omitted for better showing the pinching functionality. As can be seen, a passage 106 is closed by the first clamping portion 68, which pinches the soft tubing unit 10 in a first pinching position on the first fluid connection 80. Consequently, the first fluid connection 80 is fully closed. Further, a passage 108 (concealed) is closed by the second clamping portion 70, which pinches the soft tubing unit 10 in the sample container portion 96. Therefore, no fluid can flow from the sample container 6 to the suctioning source (not shown). By pinching the soft tubing unit 10 in the described first and second pinching positions, fluid can just flow directly from the endoscope 4 to the suctioning source (not shown), through open passage 110, bypassing the sample container 6 completely. In other words, no fluid can flow from the endoscope 6 directly to the sample container 6.

The closed passage 106 is formed by one corresponding straight portion of the pinching unit 12 moving closer to the corresponding curved portion of the tubing holder 32, via the rotation of the pinching unit 12, thereby clamping the soft tubing unit 10. Due to the omitted soft tubing unit 10, it can be seen that the closed passage 106 has the shape of a circular segment.

The closed passage 108 is formed, when the corresponding straight portion of the pinching unit 12 moves closer to the corresponding curved portion of the tubing holder 32, via the rotation of the pinching unit 12, thereby clamping the soft tubing unit 10. Due to the omitted soft tubing unit 10, it can be seen that the closed passage 108 has the shape of a circular segment.

When the pinch valve 8 is in an assembled state, a combined first and second position portion 78 of the pinching unit 12 lies outside the tubing holder 32. In other words, an inner surface of the combined first and second position portion 78 abuts against an outer surface of the tubing holder 32.

Fig. 12 is an exploded cross sectional view of the medical sampling device 2 according to the first embodiment with illustrated pinching positions 144, 146, 148. Visible are the sample container attachment element 16, the endoscope attachment element 20, the housing 28, the pinching unit 12 with the clamping claw 76, the soft tubing unit 10 and the tubing holder 32 sandwiched in-between in an assembled state. Further, Fig. 12 illustrates three X-shaped crosses, which are drawn at the pinching positions 144, 146, 148. It is mentioned, that the functionality of the medical sampling device 2 is not influenced, if the pinching positions 144, 146, 148 are slightly moved/ shifted. When the by-pass mode is used, the soft tubing unit 10 is pinched/ clamped in the first pinching position 144 and the second pinching position 146. When the sampling mode is used, the soft tubing unit 10 is pinched/ clamped in the third pinching position 148. Correspondingly, the first pinching position 144 and the second pinching position 146 are open in the sampling mode and respectively the third pinching position 148 is open in the by-pass mode. This leads in each case to just one possible fluid flow from the endoscope 4 to the suctioning source 13.

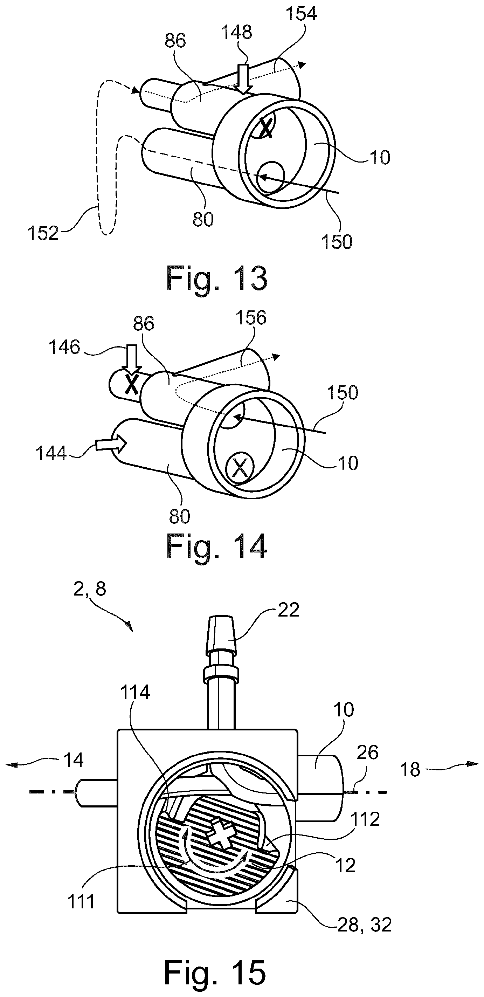

Fig. 13 is a perspective view of the soft tubing unit 10 of the medical sampling device 2 according to the first embodiment with the illustrated pinching position 148 in the sampling mode. The soft tubing unit 10 comprises the first fluid connection 80 and the second fluid connection 86. The black X-shaped cross indicate a corresponding closed fluid flow path. In the shown sampling mode, the fluid flows along a fluid way 150 through the first fluid connection 80, then enters and exits the sample container 6 (see Fig. 1) along a fluid way 152 and finally flows towards the suctioning source 13 (see Fig. 1) along a fluid way 154.

Fig. 14 shows a perspective view of the soft tubing unit 10 of the medical sampling device according to the first embodiment with illustrated pinching positions 144, 146 in the by-pass mode. The soft tubing unit 10 comprises the first fluid connection 80 and the second fluid connection 86. The black X-shaped crosses indicate corresponding closed fluid flow paths. In the shown by-pass mode, the fluid flows along the fluid way 150 through the second fluid connection 86 and then flows towards the suctioning source 13 (Fig. 1) along a fluid way 156.

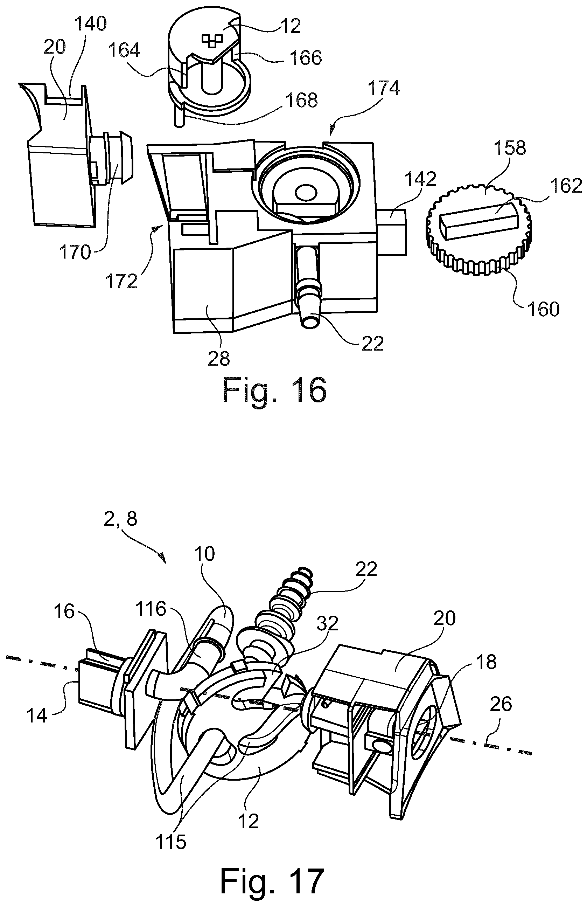

Fig. 15 shows a perspective view of the medical sampling device 2 according to a second embodiment, and Fig. 16 shows an exploded view of a variant of this embodiment. The functionality of the medical sampling device 2 is similar to the first embodiment, but in this second embodiment pinching is provided by pressing the soft tubing against a back wall as will be discussed below. However, the rotational axis of the pinching unit 12 is essentially perpendicular to the longitudinal axis 26 and is indicated by arrow 111. The rotation axis of the pinching unit 12 may be off-set relative to the axis 26 as shown, which has the advantage that assembly is facilitated. This embodiment comprises only few parts and assembly is simple, which makes it suitable for single-use medical devices where cost of manufacturing is important.

In the second embodiment, the medical sampling device 2 comprises a sample container connection interface 14, an endoscope connection interface 18, a suctioning source interface 22, a longitudinal axis 26, a pinch valve 8, wherein the pinch valve 8 comprises a soft tubing unit 10, which is pinchable by a pinching unit 12, and a tubing holder 32 as a stationary part, which is configured to hold the soft tubing unit 10. An additional stationary part, e.g. like the housing 28 in the first embodiment, is not necessary, however basically conceivable according to the second embodiment.

Further the pinching unit 12 has a mechanical actuation unit, e.g. formed as a rotatable wheel or turning knob 158 (Fig. 16).

A sampling-mode and a bypass-mode can be achieved by rotating the pinching unit 12 around a rotational axis, which is essentially perpendicular to the longitudinal axis 26 and points out of the image plane in the second embodiment of the medical sampling device 2. This rotation leads to a movement of the pinching unit 12 relative to the tubing holder 32, which gradually pinches the soft tubing unit 10 in predetermined positions. The predetermined positions are defined by the corresponding modes, namely the sampling-mode and the bypass-mode. Preferably, the rotational angle between these two modes is around 90°.

The medical sampling device 2 preferably allows just one internal degree of freedom, which is the rotation around the rotational axis.

Further, the components or parts of the medical sampling device 2 can be divided into a stationary group and a rotatable group. The stationary group comprises the tubing holder 32, the soft tubing unit 10, the sample container attachment element 16 and the endoscope attachment element 20. The rotatable group comprises the pinching unit 12. It is added that the system borders of the internal degrees of freedom do not include external degrees of freedom, such as movements of the entire medical sampling device 2 or the system or set comprising the medical sampling device 2, the endoscope 4 and the sample container 6, e.g. during use, assembly, disassembly, etc. and movements of connection elements, which occur when the endoscope 4 is connected or disconnected, when the sample container 6 is connected or disconnected, for example.

The pinching unit 12 further comprises a narrow clamping portion 112 and a wide clamping portion 114, which are essentially arranged diametrically opposed to each other. The narrow clamping portion 112 is configured to pinch the soft tubing unit 10 in a third position, which is located on the endoscope portion 94, shown in Fig. 9, allowing a sampling-mode. In the sampling-mode, fluid flow is just allowed from the endoscope 4 to the sample container 6 and from the sample container 6 to the suctioning source. The wide portion 114 is configured to pinch the soft tubing unit 10 in a first position, which is located on the first fluid connection 80, shown in Fig. 9, and in a second position, which is located on the sample container portion 96, shown in Fig. 9. In combination, by pinching the soft tubing part 10 in these two positions, the bypass-mode is enabled. In the bypass-mode, fluid flow is just allowed from the endoscope 4 directly to the suctioning source.

Fig. 16 shows an exploded perspective view of the medical sampling device 2 according to the second embodiment. The endoscope attachment element 20, the pinching unit 12, the housing 28, the suction source connection interface 22, the cover 142 and the turning knob 158 are visible.

The turning knob 158 comprises a fluted section 160 and an extrusion 162, and is adapted to be rotated in an easy way by the user. Further, the turning knob 158 is fixedly mounted on top of the pinching unit 12, so that a rotation of the turning knob 158 rotates the pinching unit 12, in order to set the sampling mode or the by-pass mode. The extrusion 162 is adapted to visually show a fluid flow direction, either to the sample container 6 or directly to the suctioning source 13 (see Fig. 1), according to the corresponding mode (sampling mode or by-pass mode).

In order to achieve the corresponding sampling mode or by-pass mode, the pinching unit 12 comprises a narrow clamping portion 164 and a wide clamping portion 166. In the sampling mode, just the narrow clamping portion 164 clamps/ pinches the soft tubing unit 10 in a third pinching position 148 as described in Fig. 12 and in the by-pass mode, the wide clamping portion 166 clamps/ pinches the soft tubing unit 10 in a first pinching position 144 and a second pinching position 146 as well as described in Fig. 12.

Further, the clamping unit 12 comprises a pin 168, which is adapted to guide the clamping unit 12 and/ or to temporarily fixate the clamping unit 12 in a rotational position, according to the desired sampling mode or by-pass mode.

The endoscope attachment element 20 is adopted to connect with its barbed end 170 to the soft tubing unit 10 and with a side opposite to the barbed end 170 to the endoscope 4 (see Fig. 1). The endoscope 4 (see Fig. 1) is fixed/ kept in place by the locking key 140.

When assembled, the endoscope attachment element 20 sits in the aperture 172 of the housing 28 and the pinching unit 12 sits in the circular aperture 174 of the housing 28. The turning knob 158 then sits atop of the pinching unit 12.

Fig. 17 shows a perspective view of the medical sampling device 2 according to a third embodiment. The functionality is similar to the first embodiment. However, a rotational axis of the pinching unit 12 is basically rotatable, advantageously allowing a broader constructional degree of freedom.

In the third embodiment the medical sampling device 2 comprises a sample container attachment element 16 having a sample container connection interface 14, an endoscope attachment element 20 having an endoscope connection interface 18, a suctioning source interface 22, a longitudinal axis 26, a pinch valve 8, wherein the pinch valve 8 comprises a soft tubing unit 10, which is pinchable by a pinching unit 12, and a tubing holder 32, which is configured to hold the soft tubing unit 10. A pinching plane is defined by abutting surfaces of the pinching unit 12 and the tubing holder 32. The soft tubing unit 10 is essentially only pinched in the pinching plane. In other words, substantially no axial distance between pinching positions exists in the third embodiment.

The soft tubing unit comprises at least three separate soft tubes 115 and tube connectors 116, which are configured to connect the separate soft tubes 115 to achieve functionally equal fluid connections as in the previous first and second embodiments.

A sampling-mode and a bypass-mode can be achieved by rotating the pinching unit 12 around the rotational axis. This rotation leads to a movement of the pinching unit 12 relative to the tubing holder 32, which gradually pinches the soft tubing unit 10 in predetermined positions. The predetermined positions are defined by the corresponding modes, namely the sampling-mode and the bypass-mode. Preferably, the rotational angle between these two modes is smaller than or equal to 90°.

The medical sampling device 2 preferably allows just one internal degree of freedom, which is the rotation around the rotational axis.

Further, the components or parts of the medical sampling device 2 can be divided into a stationary group and a rotatable group. The stationary group comprises the tubing holder 32 (and potentially a housing similar to the housing 28 of the first embodiment), the soft tubing unit 10, the sample container attachment element 16 and the endoscope attachment element 20. The rotatable group comprises the pinching unit 12. It is added that the system borders of the internal degrees of freedom do not include external degrees of freedom, such as movements of the entire medical sampling device 2 or a system or set comprising the medical sampling device 2, the endoscope 4 and the sample container 6, e.g. during use, assembly, disassembly, etc. and movements of connection elements, which occur when the endoscope 4 is connected or disconnected, when the sample container 6 is connected or disconnected, for example.

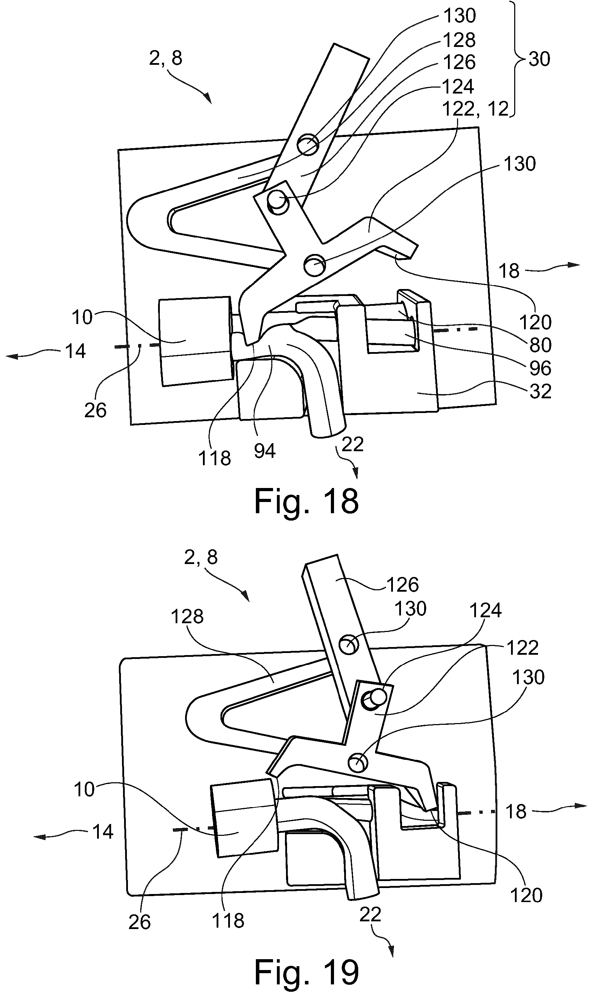

Fig. 18 shows a conceptual schematic illustration of a pinch valve prototype for a medical sampling device 2 according to a fourth embodiment in a sampling-mode. This pinch valve prototype provides a rocker switch type of operation, which may be advantageous by providing potentially user-friendly activation by shifting the actuation unit to one side or the other, which could be done with a thumb by the user with minimum effort and without changing grip on the endoscope. The pinch valve according to this embodiment may be configured to be bi-stable meaning that the pinch valve is always pinched to one side of the other, so the valve is either in bypass-mode or sampling mode.

In the fourth embodiment the medical sampling device 2 comprises a sample container attachment element 16 having a sample container connection interface 14, an endoscope attachment element 20 having an endoscope connection interface 18, a suctioning source interface 22, a pinch valve 8, wherein the pinch valve 8 comprises a soft tubing unit 10, which is pinchable by a pinching unit 12, and a tubing holder 32, which is configured to hold the soft tubing unit 10. Further, the pinching unit 12 has a mechanical actuation unit 30, which comprises a clamper 122, which acts as the pinching unit 12, an axle 124, a rocking lever 126, a spring element 128 and first and second connection interfaces 130.

The axle 124 rotatably connects the clamper 122 with the rocking lever 126, wherein the spring element is connected to the clamper 122 and the rocking lever 126 by the first and second connection interfaces 130, which preferably provide a riveted or bolted connection. The spring element 128 is configured to pull together the first and second connection interfaces 130 in a way, that a clamping force, which pinches the soft tubing unit 10 is enough to close a corresponding fluid connection within the soft tubing unit 10.

A sampling-mode and a bypass-mode can be achieved by rocking or shifting the pinching unit 12 around a rotational axis, which is essentially perpendicular to the longitudinal axis 26 and points out of the image plane in the fourth embodiment of the medical sampling device 2. The rotation leads to a movement of the pinching unit 12 relative to the tubing holder 32, which gradually pinches the soft tubing unit 10 in predetermined positions. The predetermined positions are defined by the respective modes, namely the sampling-mode and the bypass-mode.

The medical sampling device 2 preferably allows just one internal degree of freedom, which is the rotation around the rotational axis.

Further, the components or parts of the medical sampling device 2 can be divided into a stationary group and a rotatable group. The stationary group comprises the tubing holder 32, the soft tubing unit 10, the sample container attachment element 16 and the endoscope attachment element 20. The rotatable group comprises the pinching unit 12. It is added that the system borders of the internal degrees of freedom do not include external degrees of freedom, such as movements of the entire medical sampling device 2 or a system or set comprising the medical sampling device 2, the endoscope 4 and the sample container 6, e.g. during use, assembly, disassembly, etc., and movements of connection elements, which occur when the endoscope 4 is connected or disconnected, when the sample container 6 is connected or disconnected, for example.

The pinching unit 12 further comprises a narrow clamping portion 118 and a wide clamping portion 120. The narrow clamping portion 118 is configured to pinch the soft tubing unit 10 in a third position, which is located on the endoscope portion 94, allowing a sampling-mode. In the sampling-mode, fluid flow is only allowed from the endoscope 4 to the sample container 6 and from the sample container 6 to the suctioning source. The wide portion 120 is configured to pinch the soft tubing unit 10 in a first position, which is located on the first fluid connection 80, and in a second position, which is located on the sample container portion 96. In combination, by pinching the soft tubing part 10 in these two positions, the bypass-mode is allowed. In the bypass-mode, fluid flow is only allowed from the endoscope 4 directly to the suctioning source.

Fig. 19 shows a perspective view of the medical sampling device 2 according to the fourth embodiment in the bypass-mode.

As can be seen, the wide portion 120 pinches the soft tubing unit 10 in the first position, which is located on the first fluid connection 80, and in the second position, which is located on the sample container portion 96. In combination, by pinching the soft tubing unit 10 in these two positions, the bypass-mode is allowed. In the bypass-mode, fluid flow is only allowed from the endoscope 4 directly to the suctioning source.

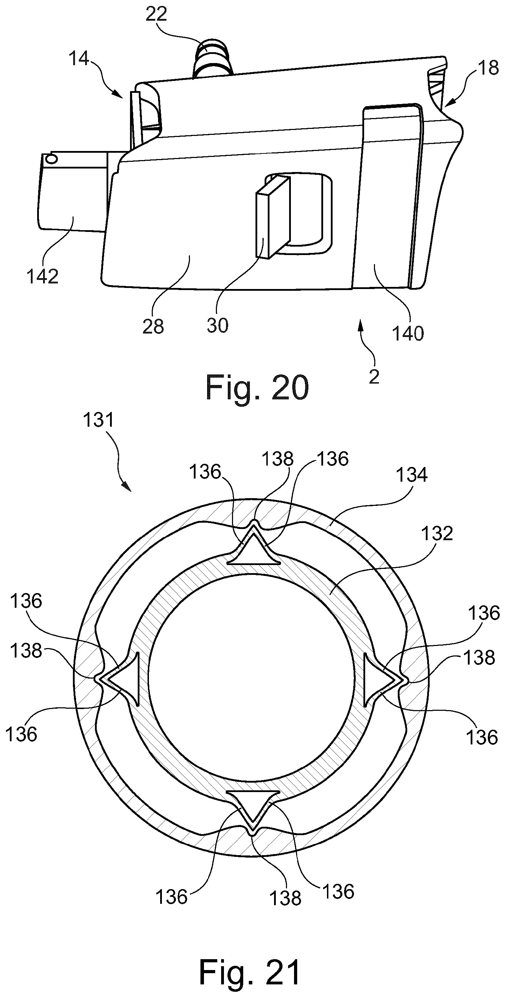

Fig. 20 shows a perspective view of the medical sampling device 2 according to the fourth embodiment. The medical sampling device 2 is in a fully assembled state and therefore compact and aesthetical appealing. In the illustrated assembled state, the housing 28, the sample container connection interface 14, the endoscope connection interface 18, the mechanical actuation unit 30, the suction source connection interface 22, the locking key 140 and the cover 142 for the sample container connection interface 14 are visible. When engaged and fully assembled (i.e. when the endoscope 4 is connected to the endoscope connection interface 18), the locking key 140 locks the endoscope 4 in the fixed position. The mechanical actuation unit 30 comprises a lever, which is adapted to be easily touched/ gripped by the user in an ergonomic way to facilitate a fast mode selection (sampling mode or by-pass mode). Further, the suction source connection interface 22 is barbed in order to securely connect to a hose. As can be seen, the cover 142 fully sheathes fluid openings of the sample container interface 14, to safeguard them from external contamination and other possible unintended influences.

Fig. 21 shows a side view of a snap-in mechanism 131. The snap-in mechanism 131 comprises a rotatable part or portion 132, a stationary part or portion 134, four spring portions 136 and four grooves 138. The grooves 138 are located within the stationary part or portion 134 and configured to accommodate the spring portions 136 in a mating-surfaces-manner. The snap-in mechanism is configured to temporarily fixate the rotatable part or portion 132 relative to the stationary part or portion 134 in such a way that an elastic deformation of the soft tubing unit 10, when being pinched, cannot initiate a rotational movement of the rotating part by an elastic restoring force of the pinched soft tubing unit 10. In a simple version there is only one spring portion and two grooves, one for bypass position, and one for the sample position, or one groove and two spring portions.

### List of reference numbers

- 2: medical sampling device
- 3: suction connector
- 4: endoscope
- 5: working channel
- 6: sample container
- 7: suction button
- 8: pinch valve
- 9: monitor
- 10: soft tubing unit
- 11: cable
- 12: pinching unit
- 13: suctioning source
- 14: sample container connection interface
- 15: insertion cord
- 16: sample container attachment element
- 17: hose
- 18: endoscope connection interface
- 20: endoscope attachment element
- 22: suctioning source connection interface/ suction source end
- 26: longitudinal axis
- 28: housing
- 30: mechanical actuation unit
- 32: tubing holder
- 34: first tubing connection
- 36: second tubing connection
- 38: tubing connection
- 40: housing endoscope opening
- 42: housing suctioning source opening
- 44: first sample container opening
- 46: second sample container opening
- 48: curved portion
- 50: straight portion
- 52: actuation unit opening
- 54: tubing holder recess
- 56: tubing holder protrusion
- 58: first tubing holder endoscope opening
- 60: second tubing holder endoscope opening
- 62: suctioning source opening
- 64: second tubing holder sample container opening
- 66: clamping unit recess
- 68: first clamping portion (first position)
- 70: second clamping portion (second position)
- 72: curved portion
- 74: straight portion
- 76: clamping claw
- 78: combined first and second position portion
- 80: first fluid connection
- 82: first endoscope end
- 84: first sample container end
- 86: second fluid connection
- 88: second endoscope end
- 90: second sample container end
- 92: suctioning source end
- 94: endoscope portion
- 96: sample container portion
- 98: suctioning source portion
- 99: common point
- 100: passage (clamped)
- 102: passage (open)
- 104: passage (open)
- 106: passage (clamped)
- 108: passage (clamped)
- 110: passage (open)
- 111: arrow
- 112: narrow clamping portion/ second engagement end/ portion
- 114: wide clamping portion/ first engagement end/ portion
- 115: soft tubes, plurality of tubes
- 116: tube connectors
- 118: narrow clamping portion/ second engagement end/ portion
- 120: wide clamping portion/ first engagement end/ portion
- 122: clamper
- 124: axle (with through hole)
- 126: rocking lever
- 128: spring element
- 130: first and second through holes
- 131: snap-in mechanism
- 132: rotating part/ portion
- 134: stationary part/ portion
- 136: spring portion
- 138: groove
- 140: locking key
- 142: cover
- 144: first pinching position
- 146: second pinching position
- 148: third pinching position
- 150: fluid way
- 152: fluid way
- 154: fluid way
- 156: fluid way
- 158: turning knob
- 160: fluted section
- 162: extrusion
- 164: narrow clamping portion
- 166: wide clamping portion
- 168: pin
- 170: barbed end
- 172: aperture
- 174: circular aperture

## Claims

1. Medical sampling device (2), in particular sampler bridge,
attachable to a suction connector (3) of an endoscope (4), to a sample container (6), and to a suctioning source (13),
operable in at least a sampling-mode and a bypass-mode,
the sampling-mode being a sample-collection-mode in which a specimen sample from the endoscope (4) is collected in the sample container (6),
the bypass-mode being a suctioning-mode in which the specimen sample is directly guided towards the suctioning source, thereby bypassing the sample container (6),
the medical sampling device (2) comprising a pinch valve (8) having at least a soft tubing unit (10) and a pinching unit (12), the pinching unit (12) being configured to pinch the soft tubing unit (10) in at least a first pinching state providing the sampling-mode and a second pinching state providing the bypass-mode.

2. The medical sampling device (2) according to claim 1 further comprising
a sample container connection interface (14) adapted for connection to the sample container (6),
an endoscope connection interface (18) adapted for connection to the endoscope (4) and
a suction source connection interface (22) adapted for connection to the suction source.

3. The medical sampling device (2) according to any one of the previous claims further comprising
a first axial end on which the endoscope (4) is attachable and
a second axial end, which is opposite the first axial end, on which the sample container (6) is attachable, wherein
a longitudinal axis (26) is formed between the first axial end and the second axial end, wherein
the suction source connection interface (22) is oriented at an angle with respect to the longitudinal axis (26), preferably substantially at a right angle.

4. The medical sampling device (2) according to any one of the previous claims further comprising
an actuation unit configured to actuate the pinching unit (12), in particular to bring the pinching unit (12) in at least the first pinching state and the second pinching state,
preferably wherein the actuation unit is a mechanical actuation unit (30), in particular is or comprises a rotatable or displaceable actuation element or portion, e.g. a lever or switch or wheel part or portion.

5. The medical sampling device (2) according to any one of the previous claims, wherein the soft tubing unit (10) is an integral part or comprises a plurality of tubes (115).

6. The medical sampling device (2) according to any one of the previous claims, wherein
the soft tubing unit (10) comprises a first fluid connection (80) having a first endoscope end (82) and a first sample container end (84), and
a second fluid connection (86) having a second endoscope end (88), a second sample container end (90) and a suction source end (22), wherein
the pinching unit (12) is configured to pinch the soft tubing unit (10) in the second pinching state providing the bypass-mode such that fluid flow is enabled between the second endoscope end (88) and the suction source end (22), and
the pinching unit (12) is configured to pinch the soft tubing unit (10) in the first pinching state providing the sampling-mode such that fluid flow is enabled from the first endoscope end (82) to the first sample container end (84), via the sample container (6) to the second sample container end (90) and from the second sample container end (90) to the suction source end (22).

7. The medical sampling device (2) according to any one of the previous claims, wherein
the pinching unit (10) is an integral part or comprises a plurality of pinching parts.

8. The medical sampling device (2) according to any one of the previous claims further comprising
a first axial end on which the endoscope (4) is attachable and
a second axial end, which is opposite the first axial end, on which the sample container (6) is attachable, wherein
a longitudinal axis (26) is formed between the first axial end and the second axial end, wherein
the pinching unit (10) is a rotating part having an axis of rotation corresponding to the longitudinal axis (26).

9. The medical sampling device (2) according to any one of the claims 1 to 7, further comprising
a first axial end on which the endoscope (4) is attachable and
a second axial end, which is opposite the first axial end, on which the sample container (6) is attachable, wherein
a longitudinal axis (26) is formed between the first axial end and the second axial end, wherein
the pinching unit (10) is a rotating part having an axis of rotation arranged essentially perpendicularly to the longitudinal axis (26).

10. The medical sampling device (2) according to claim 9, wherein
the soft tubing unit (10) comprises a first fluid connection (80) having a first endoscope end (82) and a first sample container end (84), and
a second fluid connection (86) having a second endoscope end (88), a second sample container end (90) and a suction source end (22)
the pinching unit (10) formed as the rotating part comprises a first engagement end or portion (114, 120) configured and provided to pinch the first fluid connection (80) and the second fluid connection (86), and a second engagement end or portion (112, 118) configured and provided to pinch only the second fluid connection (86), wherein
the first engagement end or portion (114, 120) has an engagement length greater than an engagement length of the second engagement end or portion (112, 118).

11. The medical sampling device (2) according to claim 10, wherein
the first engagement end or portion (114, 120) is arranged essentially diametrically opposed to the second engagement end or portion (112, 118).

12. The medical sampling device (2) according to any one of the previous claims further comprising
a locking or fixing mechanism, in particular a snap-in mechanism (131), configured and provided to lock or fixate the pinching unit (10) in the first pinching state and in the second pinching state.

13. The medical sampling device (2) according to any one of the previous claims, wherein the soft tubing unit (10) is made of silicone or TPU or rubber as material.

14. System comprising the medical sampling device (2) according to any one of the previous claims and a sample container (6), preferably also an endoscope (4).

15. The System of claim 14 comprising an endoscope (4) and a monitor (7) connectable to the endoscope.
